Europäisches Patentamt

European Patent Office

Office européen des brevets

(11) Publication number: **0 167 724**
**B1**

## (12) EUROPEAN PATENT SPECIFICATION

(45) Date of publication of patent specification: **22.06.88**

(51) Int. Cl.⁴: **C 12 Q 1/04**

(21) Application number: **85104454.5**

(22) Date of filing: **12.04.85**

(54) **Medium for malonate utilization test.**

(30) Priority: **09.05.84 JP 91064/84**

(43) Date of publication of application:
**15.01.86 Bulletin 86/03**

(45) Publication of the grant of the patent:
**22.06.88 Bulletin 88/25**

(84) Designated Contracting States:
**BE DE FR GB IT**

(56) References cited:
**EP-A-0 019 054**
**US-A-4 385 123**

**PATENT ABSTRACTS OF JAPAN, vol. 7, no. 185, 13th August 1983, (C-181) (1330); & JP-A-58-89197 (SHIJI SHIKA KOGYO K.K.) 27-05-1983**

(73) Proprietor: **TERUMO KABUSHIKI KAISHA trading as TERUMO CORPORATION**
**44-1, 2-chome, Hatagaya Shibuya-Ku**
**Tokyo 151 (JP)**

(72) Inventor: **Igarashi, Takeshi**
**31-13, 1-chome, Suwa**
**Tama-shi Tokyo (JP)**
Inventor: **Endo, Toshihiko**
**1417-1, Miyajima**
**Fuji-shi Shizuoka-ken (JP)**
Inventor: **Koshi, Isei**
**3350-1, Mannohara-Shinden**
**Fujinomiya-shi Shizuoka-ken (JP)**

(74) Representative: **Henkel, Feiler, Hänzel & Partner**
**Möhlstrasse 37**
**D-8000 München 80 (DE)**

Courier Press, Leamington Spa, England.

## 0 167 724

**Description**

### Background of the Invention

Field of the Invention

This invention relates to a medium for testing microorganisms for biochemical behavior. More particularly, this invention relates to an improved medium to be used in performing malonate utilization test on microorganisms such as enteric bacteria by the method of biochemical identification.

For administration of a proper medication to a patient of an infectious disease, it is essential to identify a pathogenic microorganism responsible for the disease, subjecting the patient to a sensitivity test, and selecting an effective medicine. In the identification of such a pathogenic microorganism, many biochemical tests are performed. One of them is a malonate utilization test. The medium of this ivention is advantageously used for the malonate utilization test.

Description of the Prior Art:

The malonate utilization test is intended to determine whether or not malonic acid can be utilized as a carbon source in the culture of a given microorganism. This test is performed by culturing the given microorganism in a culture medium containing malonate. Recently, as a culture medium for use in this test, a composition comprising proteose peptone, glucose, sodium malonate, L-tryptophane, monosodium dihydrogen phosphate, sodium chloride, bromothymol blue and purified water has been proposed (Japanese Patent Publication No. SHO 58(1983)—20263). This medium permits the time required for culture to be notably decreased as compared with the conventional countertype. Specifically, the culture time has been one to two days in the conventional medium whereas it is four to five hours in the medium under discussion, making it possible to conduct the test and find the result of the test on one same day. Depending on the kind of the microorganism under test, however, the culture performed for four to five hours in this medium does not permit the reaction to proceed sufficiently for required evaluation. In this case, the culture time must be elongated and the evaluation of the test result made on the following day. In the circumstance, the desirability of developing a medium in which any microorganism can be cultured sufficiently in a matter of four to five hours to permit clear distinction between positive and negative test of the reaction has found growing approval.

For early medication, the identification of a pathogenic microorganism is desired to be carried out as rapidly as permissible. There are, however, times when the evaluation of the test result is completed to be performed on the following day because of the convenience of the work involved in the test. In this case, the medium is desired to be such that the culture time is not rigidly specified and therefore, the culutre time may be elongated to suit the convenience of the work and, despite the elongation of the culture time, the reaction is not suffered to proceed excessively and the evaluation of the test result can be conducted accurately.

For the efficiency of work involved, many biochemical tests are frequently performed all at once by the use of a multi-well culture plate. Thus, it becomes necessary for culture times of different test items to be equalized to one another. Again in this case, the culture medium is desired not to involve any rigid limitation of culture time.

An object of this invention, therefore is to provide a novel medium for the malonate, utilization test.

Another object of this invention is to provide a medium for the malonate utilization test which does not specifically limit culture time and enables the identification of the pathogenic microorganism to be performed on the same day as the culture is carried out or on the day following the culture.

A further object of this invention is to provide a medium for malonate utilization test which permits a color reaction to produce a distinct color and enables the evaluation of test result to be effected with ease.

### Summary of the Invention

The objects described above are attained by a medium for the malonate utilization test, which comprises 0.2 to 4 g of yeast extract, 1 to 20 g of peptone, 0.3 to 3 g of glucose, 0.1 to 2 g of sodium chloride, 0.1 to 2 g of ammonium sulfate, 0.2 to 8 g of monoalkali metal dihydrogen phosphate, 4 to 40 g of a malonate and 0.01 to 0.2 g of a pH indicator, which composition assumes a pH value in the range of 6.0 to 6.5 when dissolved in 1 liter of water.

### Description of the preferred embodiment

The medium of this invention comprises yeast extract, peptone, glucose, sodium chloride, ammonium sulfate, monoalkali metal dihydrogen phosphate, a malonate and a pH indicator.

The composition of this medium comprises 0.2 to 4 g, preferably 0.3 to 3 g, of yeast extract, 1 to 20 g, preferably 1.5 to 15 g, of peptine, 0.3 to 3 g, preferably 0.4 to 2 g, of glucose, 0.1 to 2 g, preferably 0.2 to 1.9 g, of sodium chloride, 0.1 to 2 g, preferably 0.2 to 1.9 g, of ammonium sulfate, 0.2 to 8 g, preferably 0.3 to 7 g, of monoalkali metal dihydrogen phosphate, 4 to 40 g, preferably 5 to 30 g, of a malonate, and 0.01 to 0.2 g, preferably 0.06 to 0.14 g, of a pH indicator in 1 liter of water.

For the compositon of the present invention, an alkali metal salt (preferably potassium or sodium salt) of malonic acid is advantageously used as the malonate. In this invention, the medium enjoys improved

2

**0 167 724**

reactivity because it incorporates glucose. This yeast exctract is intended to promote the enzymatic reaction.

If glucose is incorporated in an excessive amount, the excess glucose decomposes itself to lower the pH value of the medium and the malonate utilization reaction consequently curbs the alkalization of the medium by positive microorganism.

The monoalkali metal dihydrogen phosphate serves as a pH adjuster. The alkali metal salt is desired to be potassium or sodium. Although this invention does not discriminate the pH indicator by its kind, the pH indicator is desired to be phenol red, bromothymol blue, bromocresol purple, cresol red, bromophenol red, chlorophenol red and the like.

The proportions of the components in the medium of this invention are critical. In particular, the proportion of the monoalkaki metal dihydrogen phosphate is fixed so that the pH value of the medium will fall in the range of 6.0 to 6.5, preferably 6.2 to 6.4, and the color of the color reaction will appear distinctly. Also the proportion of the malonate is selected to as to permit reduction in the culture time.

If the ammonium sulfate content of the medium is not sufficient, the malonate decomposition is liable to be pseudo-positive.

For the medium of the present invention, bromothymol blue proves to be the most suitable pH indicator. When this pH indicator is used, the color of the color reaction is distinct enough to permit easy discrimination between positive and negative test even when the amount of the sample size is small.

The medium of this invention is prepared for use by dissolving the components in their respective proportions in water. In recent years, it is normal for numerous tests to be performed all at once by the use of a multi-well culture plate. For the medium of this invention to be used in this manner, it is poured into the wells of the culture plate and dried to be used as dry medium. In the test, a suspension of a microorganism is dispersed in a prescribed volume to the individual wells and left standing therein to effect required culture for a prescribed length of time. By the change in color of the culture solution, the discrimination between positive test and negative test of the malonate utilization test is effected.

The medium of the present invention is used in much the same way as the conventional medium for malonate utilization test. With the medium of this invention, although the culture time can be reduced to the order of three to five hours, it is not specifically limited to that range. The cultrue may be performed for a longer period. Thus, the culture medium permits the evaluation of the test result either on the same day as the sample is taken or on the following day. The color produced in color test is so distinct as to permit easy discrimination between positive test and negative test of the reaction.

When the culture medium of this invention additionally incorporates L-tryptophane, it can be used for the tryptophane deaminase reaction test as well as for the malonate utilization test. The tryptophane deaminase reaction test is intended to detect a microorganism which deaminates tryptophane and forms indole pyruvic acid.

Now, one working example of the actual use of the medium of this invention in the malonate utilization test and referential examples of the use of the medium of this invention plus L-tryptophane in the malonate utilization test and the tryptophane deaminase test will be described below.

Examples I—V and Controls I—III

| composition of medium | Example | | | | | Control | | |
|---|---|---|---|---|---|---|---|---|
| | I | II | III | IV | V | I | II | III |
| Yeast extract (g)* | 0.5 | 0.5 | 1.0 | 0.3 | 3.0 | — | 0.5 | 0.5 |
| Peptone (g)** | 2.5 | 2.5 | 5.0 | 1.5 | 15.0 | 3.0 | 2.5 | 2.5 |
| Glucose (g) | 0.75 | 0.75 | 1.0 | 0.4 | 2.0 | 0.2 | 0.2 | 0.75 |
| Sodium chloride (g) | 1.0 | 1.0 | 0.5 | 0.2 | 1.9 | 1.0 | 1.0 | 1.0 |
| Ammonium sulfate (g) | 1.5 | 1.5 | 1.0 | 0.2 | 1.9 | — | 1.5 | 0 |
| Monosodium dihydrogen phosphate (g) | 2.0 | 2.0 | 3.0 | 0.3 | 7.0 | 5.5 | 2.0 | 2.0 |
| Sodium malonate (g) | 10.0 | 10.0 | 7.0 | 5.0 | 30.0 | 10.0 | 10.0 | 10.0 |
| Bromothymol blue (g) | 0.12 | 0.1 | 0.08 | 0.06 | 0.14 | 1.16 | 0.12 | 0.12 |
| Purified water (liter) | 1 | 1 | 1 | 1 | 1 | 1 | 1 | 1 |
| pH | 6.3 | 6.3 | 6.0 | 6.5 | 6.3 | 6.0 | 6.0 | 6.0 |

 * Yeast extract produced by Oxid Corp.
** Meat extract produced by Oxid Corp.

Culture conditions

The media of this invention and control media having the compositions as shown above were dispensed in a unit volume of 50 µl to the wells of a culture plate and dried at 40°C. Separately various microorganisms were cultured on agir culture plates at 35° to 37°C for 18 to 24 hours and suspended in 1.0 ml sterilized distilled water in a concentration of about 6 to $9 \times 10^8$ cells/ml. The suspensions were inoculated in a unit volume of 50 µl to the dry medium and, under a cover, cultured at 35° to 37°C for a prescribed length of time. By the change in color of the resultant solutions, presence or absence of the malonate decomposition reaction was evaluated. The results are shown in Table 1.

Table 1

| Microorganism | Media of Examples I-V | | Control medium I | | Control medium II | | Control medium III | |
|---|---|---|---|---|---|---|---|---|
| | 4 to 5 hours' culture | 16 to 20 hours' culture | 4 to 5 hours' culture | 16 to 20 hours' culture | 4 to 5 hours' culture | 16 to 20 hours' culture | 4 to 5 hours' culture | 16 to 20 hours' culture |
| *Enterobacter cloacae* | + + + | + + + | + + | + + + | + + + | + + + | + + + | + + + |
| *Enterobacter gergoviae* | + + + | + + + | + + | + + + | + + | + + + | + + + | + + + |
| *Klebsiella pneumoniae* | + | + + + | ± - + | + + | ± - + | + + + | + | + + + |
| *Hafnia alvei* | + + + | + + + | + | + + + | + | + + + | + + + | + + + |
| *Citrobacter diversus* | + + + | + + + | + + | + + + | + + + | + + + | + + + | + + + |
| *Salmonella arizonae* | + + + | + + + | + | + + + | + + + | + + + | + + + | + + + |
| *Morganella morganii* | - | - | - | - | - | - | ± - - | - |
| *Providencia alcalifaciens* | - | - | - | - | - | - | ± - - | - |
| *Providencia rettgeri* | - | - | - | - | - | - | ± - - | - |
| *Providencia stuartii* | - | - | - | - | - | - | ± - - | - |

+ : Color of positive test (number of +'s indicating the intensity of color)
+ - + : Intermediate color closer to color of positive test
± - - : Intermediate color closer to color of negative test
- : Color of negative test

Referential Example

The malonate utilization test and the tryptophane deaminate reaction test were carried out by following the procedure of Example 1 using the medium and the control medium each addionally incorporating L-tryptophane. The results are shown in Tables 2 and 3.

| Composition of medium | Medium of referential example | Control medium IV | Control medium V | Control medium VI |
|---|---|---|---|---|
| Yeast extract (g)* | 0.5 | — | 0.5 | 0.5 |
| Peptone (g)** | 2.5 | 3.0 | 2.5 | 2.5 |
| Glucose (g) | 0.75 | 0.2 | 0.2 | 0.75 |
| Sodium chloride (g) | 1.0 | 1.0 | 1.0 | 1.0 |
| Ammonium sulfate (g) | 1.5 | — | 1.5 | 0 |
| Monosodium dihydrogen phosphate (g) | 2.0 | 5.5 | 2.0 | 2.0 |
| Sodium malonate (g) | 10.0 | 10.0 | 10.0 | 10.0 |
| L-tryptophane (g) | 1.5 | 1.5 | 1.5 | 1.5 |
| Bromothymol blue (g) | 0.12 | 0.16 | 0.12 | 0.12 |
| Purified water (liter) | 1 | 1 | 1 | 1 |
| | Ph 6.3 | pH 6.0 | pH 6.0 | pH 6.0 |

* and ** Same as in Example.

Table 2

| Microorganism | Culture medium of the reference | | Control medium IV | | Control medium V | | Control medium VI | |
|---|---|---|---|---|---|---|---|---|
| | 4 to 5 hours' culture | 16 to 20 hours' culture | 4 to 5 hours' culture | 16 to 20 hours' culture | 4 to 5 hours' culture | 16 to 20 hours' culture | 4 to 5 hours' culture | 16 to 20 hours' culture |
| *Enterobacter cloacae* | + + + | + + + | + + | + + + | + + + | + + + | + + + | + + + |
| *Enterobacter gergoviae* | + + + | + + + | + + | + + + | + + | + + + | + + + | + + + |
| *Klebsiella pneumoniae* | + | + + + | ± – + | + + | ± – + | + + + | + | + + + |
| *Hafnia alvei* | + + + | + + + | + | + + + | + | + + + | + + + | + + + |
| *Citrobacter diversus* | + + + | + + + | + + | + + + | + + + | + + + | + + + | + + + |
| *Salmonella arizonae* | + + + | + + + | + | + + + | + + + | + + + | + + + | + + + |
| *Morganella morganii* | – | – | – | – | – | – | ± – – | – |
| *Providencia alcalifaciens* | – | – | – | – | – | – | ± – – | – |
| *Providencia rettgeri* | – | – | – | – | – | – | ± – – | – |
| *Providencia stuartii* | – | – | – | – | – | – | ± – – | – |

+ : Color of positive test (number of +'s indicating the intensity of color)
+ – + : Intermediate color closer to color of positive test
± – – : Intermediate color closer to color of negative test
– : Color of negative test

0 167 724

Table 3

| Microorganism | Medium of the reference | | Control medium IV | | Control medium V | | Control medium VI | |
|---|---|---|---|---|---|---|---|---|
| | 4 to 5 hours' culture | 16 to 20 hours' culture | 4 to 5 hours' culture | 16 to 20 hours' culture | 4 to 5 hours' culture | 16 to 20 hours' culture | 4 to 5 hours' culture | 16 to 20 hours' culture |
| *Enterobacter cloacae* | - | - | - | - | - | - | - | - |
| *Enterobacter gergoviae* | - | - | - | - | - | - | - | - |
| *Klebsiella pneumoniae* | - | - | - | - | - | - | - | - |
| *Hafnia alvei* | - | - | - | - | - | - | - | - |
| *Citrobacter diversus* | - | - | - | - | - | - | - | - |
| *Salmonella arizonae* | - | - | - | - | - | - | - | - |
| *Morganella morganii* | + + | + + | + + | + + | + + | + + | + + | + + |
| *Providencia alcalifaciens* | + + | + + | + + | + + | + + | + + | + + | + + |
| *Providencia rettgeri* | + + | + + | + + | + + | + + | + + | + + | + + |
| *Providencia stuartii* | + | + + | + | + + | + | + + | + | + + |

+      :  Color of positive test (number of +'s indicating the intensity of color)
+ - +  :  Intermediate color closer to color of positive test
+ - -  :  Intermediate color closer to color of negative test
-      :  Color of negative test

**0 167 724**

It is noted from the results of Table 1 and Table 2 that the use of the media of this invention in the malonate utilization test permit accurate identification of microorganisms without reference to length of culture time. Thus, with the medium of this invention, the evaluation of test result can be carried out either on the same day as the sample is taken or on the following day, depending on the convenience of the work involved in the test.

In contrast, in the use of control media, the color produced after 4 to 5 hours culture is not amply distinct to permit the evaluation of test result on the same day as the sample is taken.

Moreover, the fact that the medium of this invention is not rigidly restricted by the length of culture time proves highly convenient where numerous biochemical tests are carried out all at once.

**Claims**

1. A medium for malonate utilization test, comprising:
0.2 to 4 g of yeast extract,
1 to 20 g of peptone,
0.3 to 3 g of glucose,
0.1 to 2 g of sodium chloride,
0.1 to 2 g of ammonium sulfate,
0.2 to 8 g of monoalkali metal dihydrogen phosphate,
4 to 40 g of a malonate, and
0.01 t 0.2 g to a pH indicator,
which culture medium exhibits a pH value in the range of 6.0 to 6.5 when dissolved in 1 liter of water.

2. A medium according to Claim 1, which comprises:
0.3 to 3 g of yeast extract,
1.5 to 15 g of peptone,
0.4 to 2 g of glucose,
0.2 to 1.9 g of sodium chloride,
0.2 to 1.9 g of ammonium sulfate,
0.3 to 7 g of monoalkali metal dihydrogen phosphate,
5 to 30 g a malonate, and
0.06 to 0.14 g of a pH indicator,

3. A medium according to Claim 2, wherein the pH value of the medium is in the range of 6.2 to 6.4.

4. A medium according to Claim 1, wherein said monoalkali metal dihydrogen phosphate is potassium salt or sodium salt.

5. A medium according to Claim 1, wherein said malonate is an alkali metal salt.

6. A medium according to Claim 5, wherein said alkali metal salt is potassium or sodium salt.

7. A medium according to Claim 1, wherein said pH indicator is one compound selected from the group consisting of phenol red, bromothymol blue, bromocresol purple, cresol red, chlorophenol red and bromophenol red.

8. A medium according to Claim 7, wherein said pH indicator is bromothymol blue.


**Patentansprüche**

1. Medium für einen Malonatausnutzungstest, enthaltend 0,2 bis 4 g Hefeextrakt, 1 bis 20 g Pepton, 0,3 bis 3 g Glucose, 0,1 bis 2 g Natriumchlorid, 0,1 bis 2 g Ammoniumsulfat, 0,2 bis 8 g Monoalkalimetalldihydrogenphosphat, 4 bis 40 g eines Malonats und 0,01 bis 0,2 g eines pH-Indikators, wobei Kulturmedium beim Auflösen in 1 Liter Wasser einen pH-Wert im Bereich von 6,0 bis 6,5 aufweist.

2. Medium nach Anspruch 1, enthaltend 0,3 bis 3 g Hefeextrakt, 1,5 bis 15 g Pepton, 0,4 bis 2 g Glukose, 0,2 bis 1,9 g Natriumchlorid, 0,2 bis 1,9 g Ammoniumsulfat, 0,3 bis 7 g Monoalkalimetallhydrogenphosphat, 5 bis 30 g eines Malonats und 0,06 bis 0,14 g eines pH-Indikators.

3. Medium nach Anspruch 3, dadurch gekennzeichnet, daß der pH-Wert des Mediums in Bereich von 6,2 bis 6,4 liegt.

4. Medium nach Anspruch 1, dadurch gekennzeichnet, daß das Monoalkalimetalldihydrogenphosphat ein Kalium- oder Natriumsalz ist.

5. Medium nach Anspruch 1, dadurch gekennzeichnet, daß das Malonat ein Alkalimetallsalz ist.

6. Medium nach Anspruch 5, dadurch gekennzeichnet, daß das Alkalimetallsalz ein Kalium- oder Natriumsalz ist.

7. Medium nach Anspruch 1, dadurch gekennzeichnet, daß der pH-Indikator aus der Gruppe Phenolrot, Bromthymolblau, Bromkresolpurpur, Kresolrot, Chlorphenolrot und Bromphenolrot ausgewählt ist.

8. Medium nach Anspruch 7, dadurch gekennzeichnet, daß der pH-Indikator Bromthymolblau ist.

# 0 167 724

**Revendications**

1. Milieu pour le test de consommation du malonate, comprenant:
0,2 à 4 g d'extraite de levure,
1 à 20 g de peptone,
0,3 à 3 g de glucose,
0,1 à 2 g de chlorure de sodium,
0,1 à 2 g de sulfate d'ammonium,
0,2 à 8 g de dihydrogénophosphate de métal alcalin,
4 à 40 g d'un malonate, et
0,01 à 0,2 g d'un indicateur de pH,
le milieu de culture présentant une valeur de pH dans l'intervalle de 6,0 à 6,5 lorsqu'il est dissous dans 1 litre d'eau.

2. Milieu selon la revendication 1, qui comprend:
0,3 à 3 g d'extrait de levure,
1,5 à 15 g de peptone,
0,4 à 2 g de glucose,
0,2 à 1,9 g de chlorure de sodium,
0,2 à 1,9 g de sulfate d'ammonium,
0,3 à 7 g de dihydrogénophosphate de métal alcalin,
5 à g d'un malonate, et
0,06 à 0,14 g d'un indicateur de pH.

3. Mileau selon la revendication 2, dans lequel la valeur de pH du milieu est dans l'intervalle de 6,2 à 6,4.

4. Mileau selon la revendication 1, dans lequel ledit hydrogénophosphate de métal alcalin est un sel de potassium ou de sodium.

5. Mileau selon la revendication 1, dans lequel le malonate est un sel de métal alcalin.

6. Mileau selon la revendication 5, dans lequel le sel de métal alcalin est un sel de potassium ou de sodium.

7. Mileau selon la revendication 1, dans lequel l'indicateur de pH est un composé choisi dans le groupe constitué par le rouge de phénol, le bleu de bromothymol, le pourpre de bromocrésol, le rouge de crésol, le rouge de chlorophénol et le rouge de bromophénol.

8. Mileau selon la revendication 7, dans lequel l'indicateur de pH est le bleu de bromothymol.

10